Europäisches Patentamt

European Patent Office

Office européen des brevets

(11) Publication number: **0 210 588 B1**

## EUROPEAN PATENT SPECIFICATION

(45) Date of publication of patent specification: **09.12.92**

(51) Int. Cl.⁵: **C07K 7/00**, C07K 1/00, A61K 37/02, C12P 21/02

(21) Application number: **86110139.2**

(22) Date of filing: **23.07.86**

(54) **Antitumor polypeptide and a method of preparing the same.**

(30) Priority: **29.07.85 JP 167037/85**

(43) Date of publication of application:
**04.02.87 Bulletin 87/06**

(45) Publication of the grant of the patent:
**09.12.92 Bulletin 92/50**

(84) Designated Contracting States:
**AT BE CH DE FR GB IT LI LU NL SE**

(56) References cited:
**EP-A- 0 155 549**

**INT. JOURNAL CANCER, vol. 26, 1980, pages 171-176, S. TSUCHIYA et al.: "Establishment and characterization of a human acute monocytic leukemia cell line (THP-1)"**

**THE JOURNAL OF BIOLOGICAL CHEMISTRY, vol. 260, no. 4, 25th February 1985, pages 2345-2354, The American Society of Biological Chemists, Inc, US; B.B. AGGARWAL et al.: "Human tumor necrosis factor; production, purification, and characterization"**

(73) Proprietor: **Mizuno, Den'Ichi**
**Okamoto-18**
**Kamakura City, Kanagawa(JP)**

Proprietor: **Soma, Gen-Ichiro**
**1-10-21, Higashi-Tamagawa**
**Setagaya-ku, Tokyo(JP)**

(72) Inventor: **Soma, Gen-Ichiro**
**1-10-21, Higashitamagawa**
**Setagaya-ku Tokyo(JP)**
Inventor: **Mizuno, Den'ichi**
**18 Okamoto**
**Kamekura Kanagawa(JP)**
Inventor: **Shibai, Hiroshi**
**14-15 Wakamatsu-cho**
**Chigasaki Kanagawa(JP)**

(74) Representative: **von Kreisler, Alek,**
**Dipl.-Chem. et al**
**Deichmannhaus am Hauptbahnhof**
**W-5000 Köln 1(DE)**

## Description

Background of the Invention

(a) Field of the Invention

This invention relates generally to a novel antitumor polypeptide and, more specifically, to a novel human-derived antitumor polypeptide, generally classified into tumor necrosis factor(TNF), having selective toxicity against tumor(transformed, neoplastic) cells.

(b) Description of the Prior Art

As a human-derived antitumor polypeptide having a cytotoxicity against mouse L-929 fibroblast cells, there is known a polypeptide, called TNF, which is obtained from a human cell line HL-60 (ATCC240) and the amino acid sequence of which is almost entirely determined [The Journal of Biological Chemistry 260, 2345-2354 (1985)].

Such a TNF polypeptide is known to be produced by Escherichia coli transformed with a recombinant plasmid [Nature 312, 724-729 (1984, Dec. 20/27); Nature 313, 803-806 (1985, Feb. 28); Science 228, 149-154 (1985, Apr.12)]. The polypeptides produced by transformed Escherichia coli are identical with TNF disclosed in the above-described article, The Journal of Biological Chemistry 260, when deduced from the base sequence of the cloned DNA, with the exception that TNF disclosed in Nature 313 lacks the two N-terminal amino acids, i.e., Val and Arg.

Summary of the Invention

The present invention contemplates the provision of the novel antitumor polypeptide having excellent activities and a method of preparing the same.

The present inventors have obtained a novel antitumor polypeptide from differentiation inducing agent-containing culture, especially suspension culture, of human acute monocytic leukemia cells THP-1 [Int. J. Cancer 26, 171-176 (1980)] which is quite different from the above-described HL-60 human cell line.

Brief Description of the Drawing

Fig. 1 is a graph showing the NaCl concentration capable of eluting the anti-tumor activity, when an anti-tumor polypeptide-containing mixture obtained from THP-1 cells in Example 1(1) is purified with the second FPLC. And the eluate is monitored for absorbance at 280 nm and for cytotoxic activity;
Fig. 2 is reverse phase FPLC elution pattern of TNF-1 with a linear gradient of acetonitrile;

Description of the preferred embodiment(s)

In accordance with the first aspect of the present invention there is provided an antitumor polypeptide having the following N-terminal amino acid sequence:
Val-A-Ser-X-Thr-B-Thr-C-D-E-F-G-Val-Ala-His-Val-Ala-Asn wherein A represents Arg or Lys, B represents Arg or Pro, C represents Arg or Pro, D represents Ser or Lys, E represents Arg or Pro, F represents Lys or Val, G represents Phe, Pro or Ala and X is an unidentified amino acid, containing tryptic peptide fragments having the folowing amino acid sequences:

F1: Val-Val-Ala-Asn-Pro-Gln-Ala-Glu-Gly-Gln-Leu-Gln,
F2: Ala-Asn-Ala-Leu-Leu-Ala,
F3: Asn-Gln-Leu-Val-Val-X-X-X-Gly-Leu,
F4: Ile-Ala-Val-X-Tyr,
F5: Val-Asn-Leu-Leu,
F6: Glu-Thr-Pro-Glu-Gly-Ala-Glu-Ala
F7: Tyr-Glu-Pro-Ile-Tyr-Leu-Gly-Gly-X-Phe and
F8: Leu-Ser-Ala-Glu-Ile-Asn-Arg-Pro-Asp-Tyr-Leu-Asp-Phe-Ala-Glu-Ser-Gly-Gln-Val-Tyr

and having the following physical and chemical properties:
(a) molecular weight : 17400±500 (SDS electrophoresis);
(b) isoelectric point : pI 5.7±0.2 (gel isoelectric point electrophoresis);
(c) pH stability : stable at pH in the range of 6-9;

(d) temperature stability: activity is lost by about 60-70% when heated at 65°C, pH 7.0 for 1 hour;

(e) stability to protease: inactivated;

(f) physiological activity: shows no cytotoxicity against human normal cells but exhibits cytotoxicity to human tumor cells.

The above polypeptide may be produced, for example, by culture, preferably suspension culture, in a culture medium of human acute monocytic leukemia cells THP-1 [Int. J. Cancer 26 171-176 (1980)] under or after contacting them with differentiation inducing agents.

The differentiation inducing agent is a substance capable of inducing malignant monocyte cells into macrophages, monocyte cells or granuloccyte cells. Examples of the differentiation inducing agents include hemin, actinomycin D, hexamethylene-aclacinomycin A, Teleocidin, mitomycin C, Bleomycin, propionic acid, sodium acetate, cadaverine, Tunicamycin, 12-o-tetradecanoyl- phorbol-13-acetate (TPA), lymphokines such as γ-interferon, D-factor, arginase, histone HI, lipopolysaccharide (LPS), lipid A, glucocorticoid, 1d-25-dehydrooxyvitamin $D_3$, poly(I), poly(ADP-ribose), BCG and chloroquine.

The contact of the human acute monocytic leukemia cells THP-1 with the differentiation inducing agent can be effected by culture, especially suspension culture, the THP-1 cells in a culture medium. The antitumor polypeptide may be produced by culture of THP-1 cells in the continual presence of the differentiation inducing agent. After the contact of the THP-1 cells with the differentiation inducing agent, however, the cells may be transferred to another culture medium containing no such inducing agent. In the latter case, an extracellular stimulating agent may be added to the medium to which the THP-1 cells have been transferred.

The stimulating agent is a substance which serves to change the cell walls or lysozome for facilitating the discharge or ooze of endocellular macromolecules from the cells. Illustrative of suitable stimulating agents are lipopolysaccharides, lecthins and vitamin A.

The culture medium in which the THP-1 cells are cultured may be any conventional medium for culture of various animal cells. Rosewell Park Memorial Institute-1640 Medium (hereinafter referred to as RPMI-1640) is a suitable example of such a culture medium. Other media such as Dulbeccos Modified Eagle's medium, Eagle's minimum essential medium (hereinafter referred to as MEM medium) and Chick medium may also be used. While the medium may be added with fetal bovine serum (hereinafter referred to as FBS), neonate bovine serum or horse serum, the use of serum-free medium generally gives extremely good results in the separation and purification of the antitumor polypeptide.

The suspension culture of the THP-1 cells is generally performed with a cell density of 1-5 x $10^6$/ml at 35-38°C under a carbon dioxide gas stream at concentration of 4-6 %.

The differentiation inducing agent is generally added to the medium at the commencement of the culture. The extracellular stimulating agent may be added to the medium either at the commencement of the culture or when the cells are grown to a significant degree. However, the antitumor polypeptide may be sufficiently produced without the addition of the stimulating agent. Rather, it is advantageous not to use the stimulating agent because of the convenience in the purification of the antitumor polypeptide.

The antitumor polypeptide obtained by the suspension culture of THP-1 cells may be quantitatively and qualitatively analyzed in the following manner. Target cells, L-929 cells [Proc. Natl. Acad. Sci. U.S.A. 72, 3666-3670] are grown in Eagle's minimum essential medium (MEM) added with 5% fetal bovine serum until 8x$10^4$ cells are contained per 100 μl of the medium and cultured on a flat bottom plate with 96 wells under conventional culturing conditions, namely at 37°C for 2 hours under 5% $CO_2$ and 100% $H_2O$. Then, actinomycin D is added to the medium until a concentration of 1 μg/ml is reached while adjusting the amount of the culture liquid to 150 μl. This is then immediately added with 50 μl of the sample diluted with MEM. The degree of the dilution is controlled adequately to determine ED50. The L-929 cell, the volume of which becomes 200 μl, is then cultured under the same conditions as above for 18 hours. For the purpose of measuring the cell necrosis activity, the entire medium is removed and 2% methyl alcohol solution containing 0.2% crystal violet is added for fixation staining. Since the karyote cells are stained but the cells killed and freed from the bottom of the flask are not stained with the crystal violet, the cell necrosis activity may be directly measured. Thus, the color density is measured as an absorbance of OD 590 nm and compared with that of the control. The activity is expressed as unit activity per 1 ml of the sample and is defined as the degree of dilution of the sample with which 50% of the L-929 cells are alive. For example, a sample diluted with one volume of a diluent and providing ED50 is defined as having an activity of 1 unit/ml.

The polypeptide thus accumulated in the culture is purified by conventional protein purification techniques such as ion exchange chromatography using a basic anion exchanger, salting out, dialysis, gel filtration, hydrophobic chromatography, high speed molecular sieve chromatography and electrophoresis. Two or more of the above methods may generally be combined.

More specifically, as the basic anion exchanger, there may be suitably used DEAE-Sephadex A-25 or A-50, DEAE-Sepharose CL-6B, DEAE Sephamil (manufactured by Pharmacia Inc.), or an anion exchanger containing a diethylamino group, aminoethyl group or a quarternary aminoethyl group. The buffer used for the chromatography is preferably Tris-HCl or phosphate buffer with a pH of 6.0-9.0. The antitumor polypeptide-containing culture liquid is diluted with such a buffer having a concentration of as low as 0.05 M to obtain a liquid having a salt concentration of 0.1 M or less. The liquid is then contacted with the anion exchanger to allow the polypeptide to be adsorbed thereon. The elution of the antitumor polypeptide may be carried out with a salt solution such as 0.1-0.2 M NaCl or KCl solution. The polypeptide is eluted at a salt concentration of near 0.2 M. The contact of the polypeptide with the anion exchanger is desirably conducted by a column method. In the case of a large scale treatment, however, a batch method may be adopted if desired. Before subjecting the anion exchange chromatography, the sample is preferably pretreated with the use of an ultrafiltration membrane for the removal of low molecular weight substances and for improving the purification efficiency.

The solution obtained by the anion exchange chromatography is, after dialysis, subjected to gel filtration using a filtration medium such as Sephadex G-75 or G-100 (manufactured by Pharmacia Inc.), Sephacryl S-200 (manufactured by Pharmacia Inc.), Biogel P-100 (manufactured by Biorad Inc.) or Toyo Pearl HW-50 or HW-55 (manufactured by Toyo Soda Industry Co., Ltd.). The buffer used is preferably Tris-HCl or phosphate buffer having a pH of 6.0-9.0. To prevent adsorption, a salt such as NaCl may be desirably added in an amount of 0.2-0.5 M. This step generally provides 2-10 fold purification.

The antitumor polypeptide-containing solution obtained by the anion exchange chromatography may be purified by hydrophobic chromatography. In this case, Butyl Toyo Pearl 650 (manufactured by Toyo Soda Industry Co., Ltd.) etc. may be used as a medium and a salt such as ammonium sulfate or NaCl is used for the elution of the polypeptide. This step provides 5-30 fold purification and at least 80% recovery rate. The specific activity of the antitumor polypeptide after this step is $1 \times 10^6$ or more and the content of the polypeptide is 1.0% or more.

The polypeptide-containing solution purified by gel filtration is then subjected to a high performance anion exchange chromatography such as Pharmacia Fast Protein, Peptide, Polynucleotide Liquid Chromatography (FPLC) system using Mono Q HR5/5 column (a high performance anion exchange column manufactured by Pharmacia Inc.), thereby to obtain a purified sample. This step provides 5-10 fold purification and 70-90% recovery of activity. The conditions under which the high performance anion exchange chromatography is performed are the same as those for the anion exchange chromatography described previously.

The antitumor polypeptide-containing solution purified by FPLC may be further purified to homogeneous active protein by means of SDS-electrophoresis and FPLC repeated again using Mono Q HR5/5 column. By this treatment, 5-10 fold purification is attained and the specific activity is increased to $5 \times 10^8$ units per mg-protein.

However, when the purified sample which is homogeneous and pure by the criteria of SDS-electrophoresis is subjected to ion chromatography in an FPLC system using Mono Q HR5/5 column under different elution conditions, three peaks of activity are found to be eluted. Each peak is purified again on FPLC using the same elution conditions to obtain three kinds of active protein samples named TNF-1, TNF-2 and TNF-3 (eluted in this order in the previous FPLC) whose physical and chemical properties and specific activity to L-929 cells are shown in the table below.

|  | TNF-1 | TNF-2 | TNF-3 |
|---|---|---|---|
| Molecular weight | 17,400 | 17,400 | 17,400 |
| Isoelectric point | 5.7 | 5.7 | 5.7 |
| Specific activity (Unit/mg) | $3.2 \times 10^8$ | $6.0 \times 10^8$ | $4.5 \times 10^8$ |

As can be seen from the above table, there is no difference between TNF-1, TNF-2 and TNF-3 with respect to molecular weight and isotropic point but they differ in specific activity. TNF-1, TNF-2 and TNF-3 have an N-terminal amino acid sequence shown below.

|  | 1 | 2 | 3 | 4 | 5 | 6 | 7 | 8 |
|---|---|---|---|---|---|---|---|---|
| TNF-1 | Val | -Arg- | Ser- | X | - Thr | -Arg- | Thr- | Arg- (or Pro) |
| TNF-2 | Val | -Arg- (or Lys) | Ser | - X | - Thr | -Arg- | Thr- | Pro- (or Pro) |
| TNF-3 | Val | -Arg- (or Lys) | Ser | - X | - Thr | -Arg- (or Pro) | Thr- | Pro- (or Arg) |

|  | 9 | 10 | 11 | 12 | 13 | 14 | 15 | 16 |
|---|---|---|---|---|---|---|---|---|
| TNF-1 | Ser- | Arg- | Lys (or Val) | -Phe- | Val | -Ala- | His- | Val |
| TNF-2 | Ser- (or Lys) | Arg- | Lys- (or Val) | Pro- (or Ala) | Val | -Ala- | His- | Val |
| TNF-3 | Ser- (or Lys) | Pro- (or Arg) | Lys- (or Val) | Pro- (or Ala) | -Val | -Ala- | His- | Val |

In the above N-terminal amino acid sequence, the fourth amino acid expressed as X is not identifiable by a vapor-phase amino acid sequence analyzer and is not serine. There is a possibility that X is cystein which cannot be detected by this method. It is disclosed that the N-terminal amino acid sequence of the previously described TNF from human HL-60 cell line is
Val-Arg-Ser-Ser-Ser-Arg-Thr-Pro-Ser-Asp-Lys-Pro-Val-Ala-His-Val.
The amino acid sequence of TNF-1, TNF-2 and TNF-3 differs from that of TNF in that, in the former polypeptides, the fourth amino acid is not Ser, the fifth amino acid is Thr rather than Ser, and the tenth amino acid is Arg or Pro rather than Asp. Further, the twelfth amino acid of TNF-1 is Phe. Therefore, the antitumor Polypeptide according to the present invention apparently differs from known TNF obtained from the HL-60 cells in N-terminal amino acid sequence. Japanese Published Unexamined Patent Application under Disclosure No. 60-19719 discloses that the N-terminal amino acid sequence of rabbit's TNF is;
Ser-Ala-Ser-Arg-Ala-Leu-Ser-Asp-Lys-Pro-Leu-Ala-His-Val
which differs from that of TNF of HL-60 in that the former lacks the two N-terminal amino acids and has different amino acids at the fourth, seventh, eighth and thirteenth positions. Relating to the greater portion of the amino acid sequence, it says that there is no difference between TNF of rabbit and TNF of HL-60.

The antitumor polypeptide (a mixture of TNF-1, TNF-2, and TNF-3) according to the present invention which shows a single band in SDS polyacrylamide gel electrophoresis is digested with trypsin. Thus, 3.3μg of trypsin is added to 100 μl of an aqueous solution containing 100 ug of the antitumor polypeptide and the mixture is allowed to stand at 37°C and pH 8.0 for 22 hours. The digest is applied to RP318 (column for reverse phase HPLC manufactured by Biorad Inc.) for HPLC to obtain eight fragments F-1 through F-8. Each fragment is subjected to Edman degradation using amino acid sequencing analyzer (Model 470A manufactured by Applied Biosystems Inc.) and the resulting liberated phenylthiohydantoin-amino acids are analyzed by HPLC (Shimadzu LC-4A type) to determine the amino acid sequence in the conventional manner. The results are as follows.

F-1 : Val-Val-Ala-Asn-Pro-Gln-Ala-Glu-Gly-Gln-Leu-Gln
F-2 : Ala-Asn-Ala-Leu-Leu-Ala
F-3 : Asn-Gln-Leu-Val-Val-X-X-X-Gly-Leu

F-4 :     Ile-Ala-Val-X-Tyr
F-5 :     Val-Asn-Leu-Leu
F-6 :     Glu-Thr-Pro-Glu-Gly-Ala-Glu-Ala
F-7 :     Tyr-Glu-Pro-Ile-Tyr-Leu-Gly-Gly-X-Phe
F-8 :     Leu-Ser-Ala-Glu-Ile-Asn-Arg-Pro-Asp-Tyr-Leu-Asp-Phe-Ala-Glu-Ser-Gly-Gln-Val-Tyr

Since any structure deduced from the results of anlysis of the tryptic fragments F-1 through F-8 is found in the structure of TNF of HL-60, it is believed that the amino acid sequence downstream of the thirteenth amino acid from the N-terminal of each of TNF-1, TNF-2 and TNF-3 is identical with that of TNF of HL-60.

The highly purified, antitumor polypeptide according to the present invention is expected to show no side effects when dosed to human bodies and to exhibit necrosis activity exclusively to tumor cells. The purified antitumor polypeptide shows no acute or subacute toxicity when administered to mouse intraveneously. If, therefore, the polypeptide is intraveneously dosed to a terminal stage of cancer patient with metastatic lung carcinoma from kidney, the focus will disappear, contract or stop enlarging. The antitumor polypeptide according to the present invention does not exhibit cytotoxicity against human normal cells WI-38 and Flow 1000 and fetal mouse cells, but gives cytotoxic activity against human tumor cells Hela and KB and mouse tumor cell sarcoma 180 and L-1210. Further the polypeptide exhibits excellent cytotoxity against T-24 cells which are known to be insensitive to conventional TNF[Science 230, 943-945 (1985)].

The following Examples will further illustrate the present invention.

Example 1

(1) Production of Antitumor Polypeptide by suspension culture of THP-1 cells:

Into a 300 l incubator 200 l of RPM-1640 sterile medium containing 5% fetal bovine serum was poured, into which was suspended THP-1 cells [Int. J. of Cancer 26, 171-176 (1980)] in an amount so that the cellular density became $2 \times 10^5$ /ml. The cells in the suspension were incubated at 37°C for 4 days and the culture was subjected to centrifugation to collect THP-1 cells under sterile conditions. The cells were then added to 200 l of serum-free RPMI-1640 medium contained in another incubator, to which 100 ng/ml of TPA was added. The mixture was incubated under sterile conditions at 37°C for 5 days with gentle stirring (100 r.p.m.) for induction. The culture was subjected to centrifugation for the separation and removal of the cells, thereby to obtain a supernatant liquid having antitumor polypeptide activity of $1.5 \times 10^3$ units/ml. The supernatant was concentrated to 1/10 volume by means of ultrafiltration membrane (HVLP OHV20 manufactured by Millipore Inc.). Solid ammonium sulfate (65% of the saturated amount) was dissolved in the concentrate to precipitate proteins. The precipitate was separated by centrifugation (1000 r.p.m., 20 min.) and dissolved in a small amount of 0.05 M Tris-HCl buffer (pH 7.7) followed by dialysis against the same buffer at 5°C for 24 hours. After addition of equivolume of the preceeding buffer, the protein liquid was applied to DEAE-Toyo Pearl M650 column (5x40 cm) which was previously equilibrated with the same buffer. The column was washed with 1.0 l of the buffer and eluted with the same buffer containing 0.2 M NaCl.

Fractions (2.0 l) having antitumor polypeptide activity were pooled and subjected to ammonium sulfate fractionation (40-55% saturated fraction). The thus obtained precipitate was dissolved in a small amount of water and dialyzed against the preceding buffer at 5°C for 24 hours. The dialysis inner liquid was added with ammonium sulfate (40% of the saturated amount) and the solution was centrifuged for the removal of insoluble matters and subjected to hydrophobic chromatography using Butyl-Toyo Pearl 650X column (2.5 x 30 cm), previously equilibrated with 0.05 M Tris-HCl buffer containing ammonium sulfate (40% of the saturated amount), at an elution rate of 2.0 ml/min, thereby to collect a fraction having antitumor polypeptide activity. This was dialyzed against 0.05 M Tris-HCl buffer (pH 7.8). The inner liquid of the dialysis was injected on Mono Q HR5/5 column (High Performance anion exchange column) which was previously equilibrated with 50 mM Tris-HCl buffer (pH 8.5). After washing with the same buffer, the column was eluted in a stepwise manner with increased gradients of NaCl solutions (0.1, 0.15, 0.2 and 0.3 M). The antitumor polypeptide activity was eluted with 0.2 M NaCl. The eluate was then purified until a specific activity of $1 \times 10^6$ units/mg-protein was reached. This step provided 5-15 fold purification and at least 80% recovery.

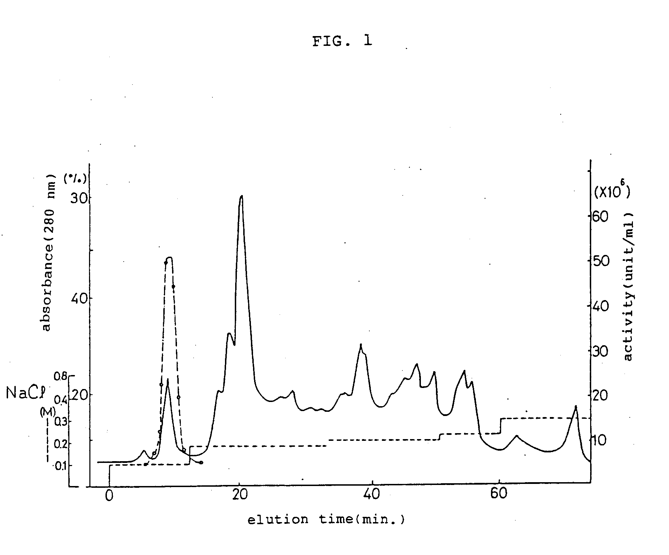

The active fractions were combined and treated again with Pharmacia FPLC (Fast Protein, Peptide, Polynucleotide Liquid Chromatography) system using Mono Q HR5/5 column under the same conditions as above. The elution pattern in the second FPLC is shown in Fig. 1 in which the ordinate represents absorbance (%) at 280 nm and abscissa represents the elution time (min). As seen from Fig. 1, the antitumor peptide activity is eluted with 0.1 M NaCl and is in good conformity with the peak monitored by absorbance at 280 nm. The active fractions were pooled and dialyzed against pure water and then lyophilized to obtain 200 $\mu$g of a purified sample having a specific activity of $5.0 \times 10^8$ units/mg-protein.

The protein sample was subjected to anion exchange column Mono Q chromatography under the conditions shown in Table 1.

Table 1

| Time (min) | Liquid A (50 mM Tris-HCl, pH 8.5) | Liquid B(1M NaCl/50 mM Tris HCl, pH 8.5) |
|---|---|---|
| 0-5 | 100% | 0% |
| 5 | 95% | 5% |
| 35 | 90% | 10% |
| 35-45 | 90% | 10% |

Three peaks were eluted at a retention time of 35, 36 and 37.8 minutes and the corresponding fractions were collected separately and named TNF-1, TNF-2 and TNF-3, respectively. Each fraction was again subjected to chromatography in the same manner as above to obtain purified protein sample which was homogeneous in the following criteria.

(2) Characterization of Antitumor Polypeptide:

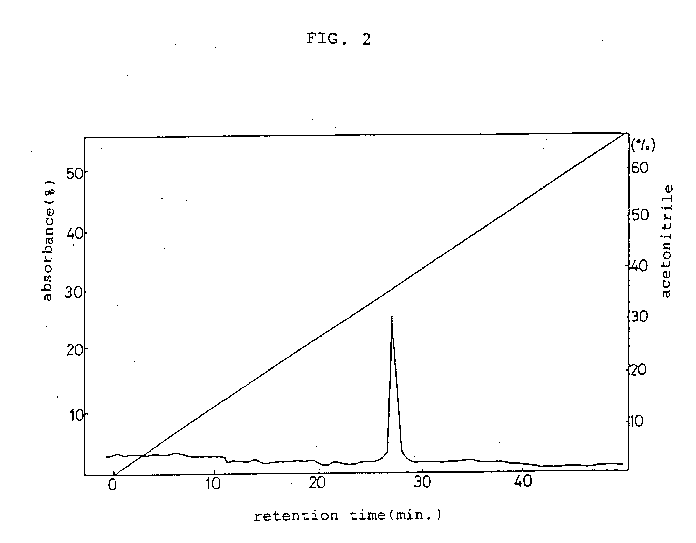

Each protein sample was subjected to reverse phase FPLC using Pro-FPC HR5/2 column (C-4 reverse phase medium manufactured by Pharmacia Inc.) using 0.1% trifluoroacetic acid as a developer with a linear gradient of 0-70% acetonitrile concentration. The elution pattern for TNF-1 is shown in Fig. 2. The TNF-1 polypeptide was eluted at an acetonitrile concentration of about 36%. No other protein peaks were detected. Similar results were obtained with respect to TNF-2 and TNF-3. Thus, each of TNF-1, TNF-2 and TNF-3 is regarded as being a single substance in the sense of the reverse phase FPLC.

Next, each protein sample was subjected to SDS-polyacryl-amide gel electrophoresis (SDS-PAGE). Thus, the sample was applied to 15.0% polyacrylamide gel containing 0.1% SDS and electrophoresed using a slab electrophoresis apparatus (Protean, 16 cm, manufactured by Biorad Inc.) at a constant current of 20 mA. The protein was detected by silver staining. In any of TNF-1, TNF-2 and TNF-3 a single band was detected at a location of 17.4 kd. No other bands were detected. It is therefore revealed that each of the samples TNF-1, TNF-2 and TNF-3 is homogeneous in the sense of the SDS-PAGE electrophoresis.

Each sample was subjected to isoelectric focusing gel electrophoresis using Ampholine polyacrylamide gel (manufactured by LKB Inc.) for the measurement of the isoelectric point (pI). Each sample was found to have an isoelectric point of 5.7.

Each sample (Ca. 10 $\mu$g) was further subjected to amino acid sequence analysis using Amino Acid Sequencing Analyzer Model 470A (manufactured by Applied Biosystems Inc.). The N-terminal amino acid sequences of the samples were determined as follows:

|  |  | 1 | 2 | 3 | 4 | 5 | 6 | 7 | 8 |
|---|---|---|---|---|---|---|---|---|---|
| TNF-1 |  | Val- | Arg- | Ser- | X - | Thr- | Arg- | Thr- | Arg -<br>(or<br>Pro) |
| TNF-2 |  | Val- | Arg-<br>(or<br>Lys) | Ser- | X - | Thr- | Arg-<br>(or<br>Pro) | Thr- | Pro - |
| TNF-3 |  | Val- | Arg-<br>(or<br>Lys) | Ser-- | X - | Thr- | Arg-<br>(or<br>Pro) | Thr- | Pro -<br>(or<br>Arg) |

|  |  | 9 | 10 | 11 | 12 | 13 | 14 | 15 | 16 |
|---|---|---|---|---|---|---|---|---|---|
| TNF-1 |  | Ser- | Arg- | Lys-<br>(or<br>Val) | Phe | -Val- | Ala | -His- | Val |
| TNF-2 |  | Ser-<br>(or<br>Lys) | Arg-<br>(or<br>Val) | Lys--<br>(or<br>Ala) | Pro - | Val- | Ala | -His- | Val |
| TNF-3 |  | Ser-<br>(or<br>Lys) | Pro-<br>(or<br>Arg) | Lys--<br>(or<br>Val) | Pro -<br>(or<br>Ala) | Val- | Ala | -His- | Val |

In the above N-terminal amino acid sequence, the fourth amino acid expressed as X is not identifiable by a vapor phase amino acid sequence analyzer and is not serine. There is a possibility that X is cystein which is unable to be detected by this method.

The antitumor polypeptide (a mixture of TNF-1, TNF-2 and TNF-3) according to the present invention which shows a single band in SDS polyacrylamide gel electrophoresis was digested with trypsin. Thus, 3.3 $\mu$g of trypsin was added to 100 $\mu$l of an aqueous solution containing 100 $\mu$g of the antitumor polypeptide and the mixture was allowed to stand at 37°C and pH 8.0 for 22 hours. The digest was applied to RP318 column (manufactured by Biorad Inc.) and reverse HPLC was employed for HLPC to obtain eight fragments F-1 through F-8. Each fragment was subjected to Edman degradation using amino acid sequencing analyzer Model 470A (manufactured by Applied Biosystems Inc.) and the resulting liberated phenylthiohydantoin-amino acids were analyzed by HPLC (Shimadzu LC-4A type) to determine the amino acid sequence in the conventional manner. The results are as follows:

F - 1 : Val-Val-Ala-Asn-Pro-Gln-Ala-Glu-Gly-Gln-Leu-Gln
F - 2 : Ala-Asn-Ala-Leu-Leu-Ala
F - 3 : Asn-Gln-Leu-Val-Val-X - X - X - Gly-Leu
F - 4 : Ile-Ala-Val- X - Tyr
F - 5 : Val-Asn-Leu-Leu
F - 6 : Glu-Thr-Pro-Glu-Gly-Ala-Glu-Ala
F - 7 : Tyr-Glu-Pro-Ile-Tyr-Leu-Gly-Gly-X-Phe
F - 8 : Leu-Ser-Ala-Glu-Ile-Asn-Arg-Pro-Asp-Tyr-Leu-Asp-Phe-Ala-Glu-Ser-Gly-Gln-Val-Tyr

Experimental Example 1

T24 cells were suspended in MEM-medium supplemented by 10 w/w% FCS and seeded in a Limbro 96 well microfilter plate in an amount of $1 \times 10^4$/200 $\mu$l/well and cultured at 37°C for 48 hours in the presence of 0.5% $CO_2$. Thereafter, the supernatant of THP-1 culture product 1% of purity, $5 \times 10^5$ units/ml, was added in amounts of 10,000 units/ml, 1,000 units/ml, 100 units/ml and 0 units/ml respectively to the medium

containing 0.1 $\mu$g/ml of actinomycine D and the culture was continued at 37°C for 16 hours in the presence of 0.5% $CO_2$. Then, 10$\mu$Ci/ml of $^3$H-thymidine was added to respective culture mixtures. After 8 hour culture, $^3$H-thymidine taken into the cells was counted by a liquid scintillation counter for the determination of the antitumor activity. The less the taken-up amount is the higher the antitumor activity is. The results are shown in Table 9. The respective value is an average of four experimental values. The value in parentheses show the rate to the amount of when only actinomycin D was dosed and THP-1 culture product was not dosed.

Table 9

| THP-1 culture product (unit/ml) | Amount of $^3$H-thymidine take-up of T24 Cells |
|---|---|
| 0 | 1222 |
| 100 | 920(75%) |
| 1,000 | 728(59%) |
| 10,000 | 472(39%) |

THP-1 cells used in the invention was described in "Int. J. Cancer 26, p.171-176(1980)". The present inventors have accomplished the invention with the cells released from the author of the paper. The cells were also described in "Proc. Natl. Acad. Sci., U.S.A. 80, 5397-5401(1983)" and "Cancer Research 42, 484-489(1982)"[It says that the cells were got from Dr. Rovera]. The cell can easily be obtained by experts and the present inventors will give the cells to any experts.

As will be appreciated from the foregoing, the novel antitumor polypeptide according to the present invention obtained by suspension-culture of THP-1 cells does not exhibit cytotoxicity to human normal cells but gives cytotoxicity to human tumor cells. The antitumor polypeptide may be prepared by a non-expensive serum-free medium. The novel DNA synthesized by a method according to the present invention can express novel antitumor polypeptides which show no cytotoxicity to normal cells but exhibit cytotoxicity to human tumor cells. Some of the novel antitumor polypeptides exhibit cytotoxicity to T-24 cells, which are insensitive to conventional TNF, and against primary culture cells which are obtained from metastasis focus of a patient with rhabdomyo sarcoma from Mullerrian duct and which show resistance to any anti-tumor chemotherapeutic agent.

**Claims**

1.  An antitumor polypeptide having the following N-terminal amino acid sequence:
    Val-A-Ser-X-Thr-B-Thr-C-D-E-F-G-Val-Ala-His-Val-Ala-Asn wherein A represents Arg or Lys, B represents Arg or Pro, C represents Arg or Pro, D represents Ser or Lys, E represents Arg or Pro, F represents Lys or Val, G represents Phe, Pro or Ala and X is an unidentified amino acid, containing tryptic peptide fragments having the folowing amino acid sequences:
    F1:    Val-Val-Ala-Asn-Pro-Gln-Ala-Glu-Gly-Gln-Leu-Gln,
    F2:    Ala-Asn-Ala-Leu-Leu-Ala,
    F3:    Asn-Gln-Leu-Val-Val-X-X-X-Gly-Leu,
    F4:    Ile-Ala-Val-X-Tyr,
    F5:    Val-Asn-Leu-Leu,
    F6:    Glu-Thr-Pro-Glu-Gly-Ala-Glu-Ala
    F7:    Tyr-Glu-Pro-Ile-Tyr-Leu-Gly-Gly-X-Phe and
    F8:    Leu-Ser-Ala-Glu-Ile-Asn-Arg-Pro-Asp-Tyr-Leu-Asp-Phe-Ala-Glu-Ser-Gly-Gln-Val-Tyr
    and having the following physical and chemical properties:
    (a) molecular weight : 17400±500 (SDS electrophoresis);
    (b) isoelectric point : pI 5.7±0.2 (gel isoelectric point electrophoresis);
    (c) pH stability : stable at pH in the range of 6-9;
    (d) temperature stability: activity is lost by about 60-70% when heated at 65°C, pH 7.0 for 1 hour;
    (e) stability to protease: inactivated;
    (f) physiological activity: shows no cytotoxicity against human normal cells but exhibits cytotoxicity to human tumor cells.

2.  A method of preparing the antitumor polypeptide according to claim 1 which comprises culturing human myeolocytic leukemia cells THP-1 in the presence of a differentiation inducing agent; or after contact with said agent.

3. A method as claimed in claim 2 wherein the cell culture is a suspension culture.

4. A method as claimed in claim 2 wherein the differentiation inducing agent is a substance capable of inducing malignant monocyte cells into macrophages, massive monocyte cells.

5. A method as claimed in claim 4 wherein the differentiation inducing agent is one or more selected from the group consisting of hemin, actinomycin D, hexamethylene aclacinomycin A, Teleocidin, mitomycin C, Bleomycin, propionic acid, sodium acetate, cadaverine, Tunicamycin, 12-o-tetradecanoylphorbol-13-acetate (TPA), a lymphokyne such as $\gamma$-interferon, D-factor, arginase, histone HI, lipopolysaccharide (LPS), lipid A, glucocorticoid, 1d-25-dehydroxyvitamin $D_3$ , poly(I), poly(ADP-ribose), BCG and chloroquine.

**Patentansprüche**

1. Antitumor-Polypeptid mit der folgenden N-terminalen Aminosäure-Sequenz:
   Val-A-Ser-X-Thr-B-Thr-C-D-E-F-G-Val-Ala-His-Val-Ala-Asn, worin
   
   A       Arg oder Lys darstellt,
   
   B       Arg oder Pro darstellt,
   
   C       Arg oder Pro darstellt,
   
   D       Ser oder Lys darstellt,
   
   E       Arg oder Pro darstellt,
   
   F       Lys oder Val darstellt,
   
   G       Phe, Pro oder Ala darstellt und
   
   X       eine nicht identifizierte Aminosäure ist,
   
   enthaltend Tryptin-Peptid-Fragmente mit den folgenden Aminosäure-Sequenzen:
   
   F1:      Val-Val-Ala-Asn-Pro-Gln-Ala-Glu-Gly-Gln-Leu-Gln,
   
   F2:      Ala-Asn-Ala-Leu-Leu-Ala,
   
   F3:      Asn-Gln-Leu-Val-Val-X-X-X-Gly-Leu,
   
   F4:      Ile-Ala-Val-X-Tyr,
   
   F5:      Val-Asn-Leu-Leu,
   
   F6:      Glu-Thr-Pro-Glu-Gly-Ala-Glu-Ala
   
   F7:      Tyr-Glu-Pro-Ile-Tyr-Leu-Gly-Gly-X-Phe, und
   
   F8:      Leu-Ser-Ala-Glu-Ile-Asn-Arg-Pro-Asp-Tyr-Leu-Asp-Phe-Ala-Glu-Ser-Gly-Gln-Val-Tyr
   
   und mit den folgenden physikalischen und chemischen Eigenschaften:
   
   (a) Molekulargewicht: 17 400 ± 500 (SDS-Elektrophorese);
   
   (b) Isoelektrischer Punkt: pI 5,7 ± 0.2 (Gel-Elektrophorese, isoelektrischer Punkt);
   
   (c) pH-Stabilität: stabil bei einem pH-Wert im Bereich 6-9;
   
   (d) Temperatur-Stabilität: Activität geht zu etwa 60-70% verloren, wenn es 1 h bei pH 7,0 auf 65 ° C erhitz wird;
   
   (e) Stabilität gegen Protease: inaktiviert;
   
   (f) physiologische Aktivität: zeigt keine Cytotoxizität gegen normale menschliche Zellen, zeigt jedoch Cytotoxizität gegen Human-Tumor-Zellen.

2. Verfahren zur Herstellung des Antitumor-Polypeptids nach Anspruch 1, umfassend das Kultivieren menschlicher myelocytischer Leukämie-Zellen THP-1 in Gegenwart eines die Differenzierung induzierenden Mittels oder nach dem Kontakt mit einem solchen Mittel.

3. Verfahren nach Anspruch 2, worin die Zellkultur eine Suspensionskultur ist.

4. Verfahren nach Anspruch 2, worin das die Differenzierung induzierende Mittel eine Substanz ist, die befähigt ist, maligne Monocyten-Zellen zu Makrophagen, massiven Monocyten-Zellen zu differenzieren.

5. Verfahren nach Anspruch 4, worin das die Differenzierung induzierende Mittel eine oder mehrere Substanz/en ist/sind, das/die aus der aus Hämin, Actinomycin D, Hexamethylen-aclacinomycin A, Teleocidin, Mitomycin C, Bleomycin, Propionsäure, Natriumacetat, Cadaverin, Tunicamycin, 12-o-Tetradecanoylphorbol-13-acetat (TPA), einem Lymphokin wie $\gamma$-Interferon, D-Factor, Arginase, Histon HI, Lipopolysaccharid (LPS), Lipid A, Glucocorticoid, 1d-25-Dehydroxyvitamin $D_3$ , poly(I), poly(ADP-ribose), BCG und Chloroquin bestehenden Gruppe ausgewählt ist/sind.

## Revendications

1. Polypeptide antitumoral ayant la séquence d'acides aminés N-terminale suivante :
   Val-A-Ser-X-Thr-B-Thr-C-D-E-F-G-Val-Ala-His-Val-Ala-Asn où A représente Arg ou Lys, B représente Arg ou Pro, C représente Arg ou Pro, D représente Ser ou Lys, E représente Arg ou Pro, F représente Lys ou Val, G représente Phe, Pro ou Ala, et X est un acide aminé non identifié, contenant des fragments peptidiques tryptiques ayant les séquences d'acides aminés suivantes :
   F1 :    Val-Val-Ala-Asn-Pro-Gln-Ala-Glu-Gly-Gln-Leu-Gln,
   F2 :    Ala-Asn-Ala-Leu-Leu-Ala,
   F3 :    Asn-Gln-Leu-Val-Val-X-X-X-Gly-Leu,
   F4 :    Ile-Ala-Val-X-Tyr,
   F5 :    Val-Asn-Leu-Leu
   F6 :    Glu-Thr-Pro-Glu-Gly-Ala-Glu-Ala
   F7 :    Tyr-Glu-Pro-Ile-Tyr-Leu-Gly-Gly-X-Phe et
   F8 :    Leu-Ser-Ala-Glu-Ile-Asn-Arg-Pro-Asp-Tyr-Leu-Asp-Phe-Ala-Glu-Ser-Gly-Gln-Val-Tyr
    et ayant les propriétés physiques et chimiques suivantes :
   (a) masse moléculaire : 17 400 ± 500 (électrophorèse SDS) ;
   (b) point isoélectrique : pI 5,7 ± 0,2 (électrophorèse sur gel au point isoélectrique) ;
   (c) stabilité du pH : stable à pH compris entre 6-9 ;
   (d) stabilité à la température : l'activité tombe d'environ 60 à 70 % après chauffage à 65°C pendant 1 heure à pH 7,0 ;
   (e) stabilité aux protéases : inactivé ;
   (f) activité physiologique : ne présente aucune cytotoxicité vis-à-vis des cellules normales humaines mais présente une cytotoxicité vis-à-vis des cellules tumorales humaines.

2. Procédé de préparation du polypeptide antitumoral selon la revendication 1, qui consiste à cultiver des cellules de la leucémie myélocytique humaine THP-1 en présence d'un agent inducteur de différenciation ; ou après contact avec cet agent.

3. Procédé selon la revendication 2, dans lequel la culture cellulaire est une culture en suspension.

4. Procédé selon la revendication 2, dans lequel l'agent inducteur de différenciation est une substance à même d'induire des cellules monocytaires malignes dans des macrophages, des cellules monocytaires massives.

5. Procédé selon la revendication 4, dans lequel l'agent inducteur de différenciation est constitué d'un ou plusieurs agents choisis dans l'ensemble comprenant l'hémine, l'actinomycine D, l'hexaméthylène-aclacinomycine A,la téléocidine, la mitomycine C, la bléomycine, l'acide propionique, l'acétate de sodium, la cadavérine, la tunicamycine, l'acétate de 12-o-tétradécanoylphorbol-13 (TPA), une lymphokyne telle que le gamma-interféron, le facteur D, l'arginase, l'histone HI, les lipopolysaccharides (LPS), le lipide A, les glucocorticoïdes, la 1d-25-déshydroxyvitamine $D_3$, le poly(I), le poly(ADP-ribose), le BCG et la chloroquine.

FIG. 1

FIG. 2

retention time(min.)